# EUROPEAN PATENT SPECIFICATION

(11) **EP 0 955 929 B1**
(45) Date of publication and mention of the grant of the patent: **03.01.2007**
(21) Application number: 96941352.5
(22) Date of filing: 13.11.1996
(51) Int. Cl.: A61B 17/70

(54) **A VARIABLE LENGTH AND ANGLE CROSS-LINK DEVICE**
KREUZVERBINDUNG MIT VARIABELER LÄNGE UND WINKEL
DISPOSITIF DE LIAISON A LONGUEUR ET ANGLES VARIABLES

(30) Priority: 15.04.1996 US 632561
(43) Date of publication of application: 17.11.1999
(73) Proprietor: Fastenetix, L.L.C., Summit, NJ 07901 (US)
(72) Inventor: ERRICO, Joseph, P., Far Hills, NJ 07931 (US); ERRICO, Thomas, J., Summit, NJ 07901 (US); RALPH, James, D., Oakland, NJ 07436 (US); CORIN, James, Englewood, CO 80112 (US)
(74) Representative: McCall, John Douglas
(86) International application number: PCT/US1996/018266
(87) International publication number: WO 1997/038640

(56) References cited:
- EP-A- 0 383 992
- US-A- 5 147 360
- US-A- 5 261 907
- US-A- 5 439 463

## Description

### Background of the Invention

### 1. Field of the Invention:

This invention relates generally to a mechanical cross-link device for use with dual rod orthopaedic implant apparatus. More particularly, this invention relates to a novel device which is fixed to each rod dual rod implant apparatus, and maintains and enhances the rigidity of the apparatus along a direction which is substantially transverse to the customary vertical orientation of the rods.

### 2. Discussion of the Prior Art:

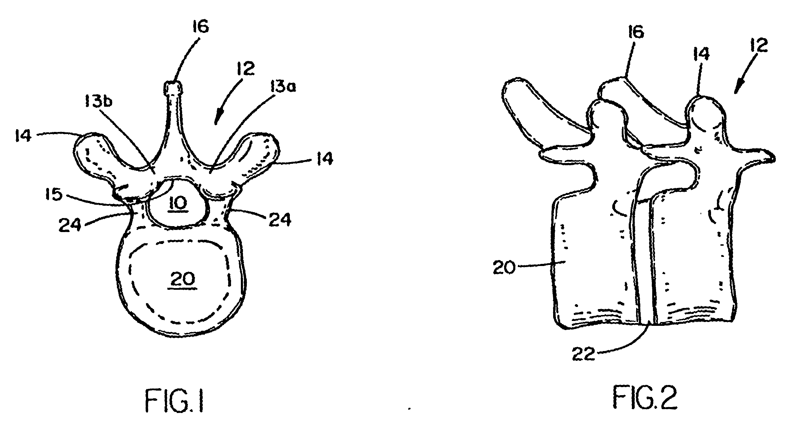

The bones and connective tissue of an adult human spinal column consist of an upper portion (the cervical, thoracic, and lumbar regions) having more than 20 discrete bones, and a lower portion which consists of the sacral bone and the coccygeal bodies. The bones of the upper portion are generally similar in shape, as will be more fully described hereinbelow with respect to Figures 1, 2 and 3. For the purpose of describing this invention, the sacral bone shall be distinguished from the spinal column; the spinal column, therefore, comprising for the purposes of this description, only the cervical, thoracic, and lumbar vertebrae.

The vertebrae vary in size, but are each similarly coupled to adjacent bones by a tri-joint complex. The tri-joint complex consists of an anterior disc and the two posterior facet joints, the anterior discs of adjacent bones being cushioned by cartilage spacers referred to as intervertebral discs. Referring now to Figures 1, 2 and 3, top, lateral, and posterior views of a typical vertebral bones of the spinal column are shown. The spinal cord is housed in the central canal 10, protected from the posterior side by a shell of bone called the lamina 12. The lamina 12 has three large protrusions, two of these extend laterally from the side ends thereof and are referred to as the transverse processes 14. The third extends back and down from the center of the lamina and is called the spinous process 16. The lamina 12 defines an arched shape about the posterior of the spinal cord, the arched shape having lateral portions 13a,13b which are generally straight, and which meet beneath the spinous process at a curved surface 15.

The anterior portion of the spine comprises a set of generally cylindrically shaped bones which are stacked one on top of the other. These portions of the vertebrae are referred to as the vertebral bodies 20, and are each separated from the other by the intervertebral discs 22. Pedicles 24 are bone bridges which couple the anterior vertebral body 20 to the corresponding lamina 12 and posterior elements 14,16.

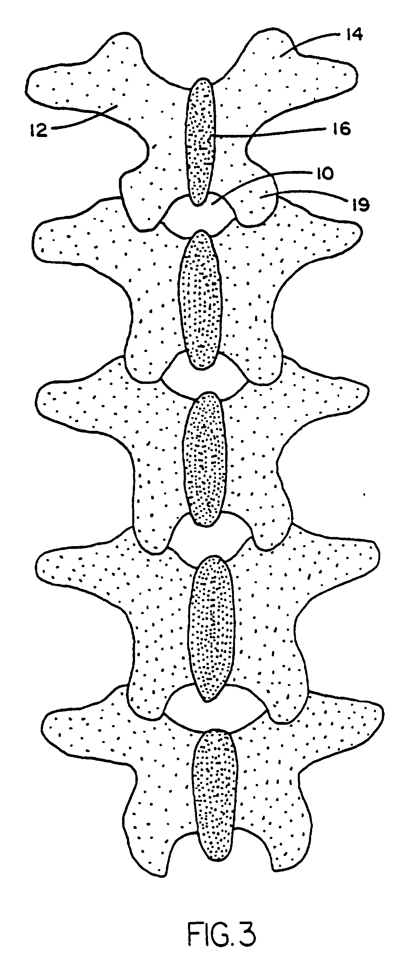

Referring specifically to Figure 3, the stacking of vertebrae is shown from the posterior. From the posterior, each vertebra is coupled to the one above and below via facet joints 19 on either side of an opening into the spinal canal 10.

In its entirety, the spinal column is highly complex in that it houses and protects critical elements of the nervous system which have innumerable peripheral nerves and arterial and venous bodies in close proximity. In spite of these complexities, the spine is a highly flexible structure, capable of a high degree of curvature and twist through a wide range of motion. Genetic or developmental irregularities, trauma, chronic stress, tumors, and disease, however, can result in spinal pathologies which either limit this range of motion, or which threaten the critical elements of the nervous system housed within the spinal column. A variety of systems have been disclosed in the art which achieve this immobilization by implanting artificial assemblies in or on the spinal column.

A variety of systems have been disclosed in the art which achieve this immobilization by implanting artificial assemblies in, or on, the spinal column. These assemblies may be classified by their position relative to the spine, as anterior, posterior, or lateral implants. Anterior and lateral assemblies generally comprise short structures which support only a few adjacent vertebral bodies. Conversely, posterior implants often comprise pairs of elongate vertically aligned rods for stabilizing both short and long segments of the spine. Such posterior rods are coupled to the back of the spinal column via hooks which slip under the lamina, means for attaching to the transverse process, and/or by screws which are inserted through the pedicle bone. In order to provide enhanced torsional rigidity, these apparatuses generally include cross-linking devices which couple the rods together transverse to the axis (vertical axis) of the apparatuses.

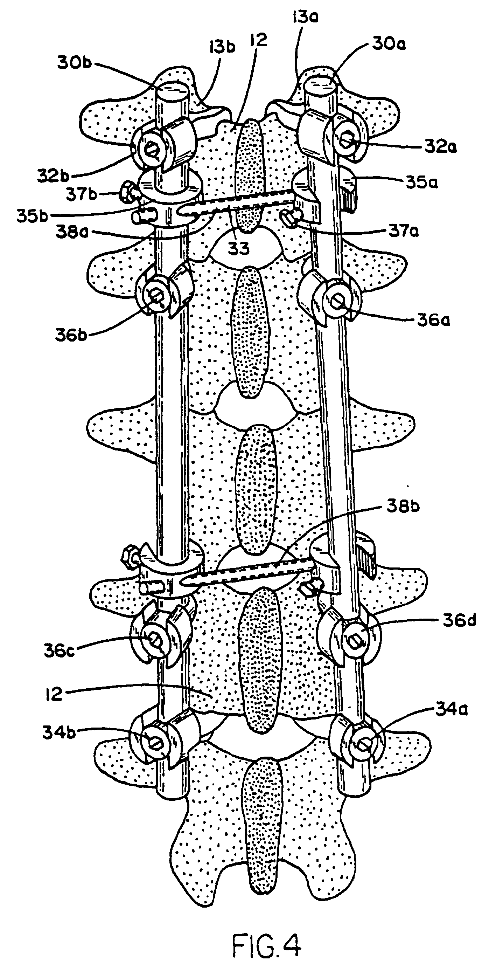

Referring now to Figure 4, U.S. Patent 5,005,562 to Cotrel teaches such a dual rod apparatus which includes a pair of rods 30a,30b, which are each coupled to the spine via hooks 32a,34a and 32b,34b, respectively, as well as pedicle screws 36a,36d and 36b,36c, respectively. The rods 30a,30b are further stabilized by cross-link devices 38a,38b. These cross-link devices 38a,38b each include a pair of U-shaped gripping element 35a,35b which may receive the rod 30a,30b respectively. Each of the gripping elements includes a first threaded hole which extends from the outer lateral surface into the inner surface of the U-shaped rod receiving region. The gripping elements 35a,35b are fixed to the rods 30a,30b by set screws 37a,37b which are positioned in the first holes such that tightening of the set screws locks the rod 30a,30b in the gripping element. The gripping elements 35a,35b are coupled together by a threaded rod 33 which permits the gripping elements to be selectively spread or brought closer together, in accordance with the relative position of the rods 30a,30b. The threaded rod 33 extends through a second set of threaded holes in the gripping elements 35a,35b.

The bulkiness of each of the gripping elements 35a,35b, required so that it may receive the threaded rod 33, is difficult for the surgeon to use easily under operative conditions. The size of the gripping elements, and the relative position of the set screws often cause substantial difficulty with respect to the tightening of same because of their positions relative to the operative access. This bulkiness also reduces available bone graft surface area, which is critical for a successful fusion and long term immobilization. In addition, in order for a surgeon to selectively vary the spread of the gripping elements 35a, 35b, one of the gripping elements must be rotated relative to the other, thus requiring the cross-link to be removed (loosening the set screws and withdrawing the device entirely from the operative site). This is particularly burdensome with respect to providing the surgeon with the ability to apply an inward force to the rods 30a, 30b as the spread may not be varied *in situ.*

US-A-5439 463 (The preamble of claim 1 is based on this document) discloses a spinal clamping device including a spinal clamping member, a clamping adjusting member, a distance adjusting bar, and a distance adjusting and fastening member.

EP-A-383 992 disclosed an apparatus comprising a pair of corrective devices for a spinal column. A member is extendable between the corrective devices in a direction transverse to the longitudinal central axes of the corrective devices. A first portion of the member is connected with one of the corrective devices and a second portion of the member is connected with the other of the corrective devices.

### SUMMARY OF THE INVENTION

According to the present invention there is provided a variable length cross-link device for use with orthopaedic rod apparatuses having a plurality of rods, comprising:
a first rod hooking element having first and second ends, and means by which said first end is coupleable to a first rod, said second end comprising a narrow extending portion;
a second rod hooking element having first and second ends, and means by which said first end is coupleable to a second rod, said second end comprising a wide flat extending portion;
first means for maintaining said narrow extending portion of said first rod hooking element in slidable relation to the wide flat extending portion of said second rod hooking element;
second means for selectively compressing a portion of said narrow extending portion against said wide flat extending portion of said second rod hooking element to crush-lock the two elements together, therein providing for selective variation in the overall length of said cross-link device, characterized in that:-
   said first means also maintains said narrow extending portion of said first rod hooking element in angulable relation to the wide flat extending portion of said second rod hooking element,
   thereby providing for selective variation in the angulated conformation of said cross link device.

The present invention may be practiced in a variety of different embodiments; the several enclosed herein being representative of preferred ones of the invention.

By use of the present invention one or more of the following may be achieved:
(i) a new and novel cross-link device which provides a less bulky profile, therein providing increased area for bone grafting.
(ii) a cross-link device which provides the surgeon with the ability to lock the device to the rods more easily than prior cross-link devices.
(iii) a cross-link device which provides the surgeon with the ability to vary the spread of the rod gripping portions *in situ,* so that in doing so, the surgeon is not required to withdraw the device from the patient.
(iv) a cross-link device which permits the surgeon to use the variable spread of the device to impart an inward force relative to the two rods, which is a desirable feature for the purposes of enhanced rotational stability.

Each of the embodiments described herein includes a pair of elements designed to attach to one of two rods in a dual rod apparatus. In a first class of embodiment, these elements are attached to the rod directly; in the second class, they are coupled to the pedicle screw. In each case, it is desirable that they be formed of a substantially flat rigid material, for example medical grade steel or titanium. In the first class, each of the pair has a first end which is curvate so as to receive therein and/or seat against and hook to the lateral outside surface of a first rod of a dual rod apparatus, and a second flat end. With respect to these curvate ends of the rod hooking elements, each includes a through hole for receiving a set screw. The set screw positioned therein is designed to be threadably tightened downward onto the rod against which the first end is seated, therein locking the rod and element together. In the second class of embodiments, the first ends of each element form loops which seat around the tops of laterally opposed pedicle screws such that they may be secured in conjunction with the final securement of the rods in their respective pedicle screws.

More particularly with respect to a general feature of each of these embodiments, the two rod hooking elements are designed to be coupled together by a threaded post, which shall be described more fully hereinafter. In a first aspect, the first of the two rod hooking elements comprises a narrowed second end, this end being alternatively either flat or cylindrical. In addition, it may be desirable for the surface of this second rod hooking end to have a grooved or roughened conformation, for example a diamond knurling.

With respect to the other of the rod hooking elements of the pair which comprise this first embodiment, the second end of this element is flat and substantially wider than a second end of the first element. The second end of the second rod hooking element further comprises a through hole which is wide enough to receive the above-introduced, threaded post, which is described more fully hereinbelow. In addition, the undersurface of the flat end of the second rod hooking element may be correspondingly roughened with, for example a diamond knurl.

The coupling of these two rod hooking elements is achieved by the incorporation of the above-mentioned threaded post. This threaded post is generally cylindrical and comprises a widened base, an intermediate portion having a passageway defined therethrough which is transverse to the axis of the post, and a threaded upper portion. The second end of the first rod hooking element is inserted through the passageway. The threaded upper portion of the post is positioned through the hole in the second end of the second rod hooking element (such that the knurled surfaces of the second ends of the first and second elements are in contact with one another, if such knurling has been included). A top locking nut is then threaded onto the upper portion of the post. Prior to the tightening of the nut, the second end of the first rod hooking element may slide relative to the flat portion of the second, so that the rod securing portions may be spread or drawn together and/or angulated relative to one another within the plane which is orthogonal to the axis of the threaded post, in accordance with the positioning of the rods. In the embodiment wherein the second end of the first rod hooking element is cylindrical, it is also possible for the elements to be axially rotated relative to one another as well. Tightening of the nut down onto the top surface of the second flat portion causes the post to be drawn upwards until the roughened surfaces of the first and second flat portions seat and lock to one another under compressive force.

### Brief Description of the Drawings

The invention will now be further described by way of example with reference to the accompanying drawings, in which:-
Figure 1 is a top view of a vertebra of the human spine;
Figure 2 is a side view of a sequence of vertebrae of the human spine;
Figure 3 is a posterior view of a sequence of vertebrae of the human spine;
Figure 4 is a posterior view of a dual rod apparatus of a prior art instrumentation as set forth in U.S. Patent 5,005,562 to Cotrel, including cross-link devices;
Figure 5 is a side perspective view of a first rod hooking element which is an aspect of the present invention;
Figure 6 is a side view of a set screw which is another aspect of the present invention;
Figure 7 is a side view of a post which is also used in conjunction with an embodiment of the present invention;
Figure 8 is a side view of a top locking nut which threadably mates to an upper portion of the post of Figure 7;
Figure 9 is a side perspective view of a second rod hooking element which is an aspect of the present invention;
Figure 10 is a side perspective view of a fully assembled embodiment of the present invention comprising all of the elements illustrated in Figures 5-9;
Figure 11 is a side perspective view of an alternative first element;
Figure 12 is a side perspective view of an alternative second element;
Figure 13 is a side perspective view of an alternative post member; and
Figure 14 is a side perspective view of a fully assembled alternative embodiment of the present invention which includes the elements illustrated in Figures 8 and 11-13.

### Detailed Description of the Preferred Embodiment

While the present invention will be described more fully hereinafter with reference to the accompanying drawings, in which particular embodiments and methods of implantation are shown, it is to be understood at the outset that persons skilled in the art may modify the invention herein described while achieving the functions and results of this invention. Accordingly, the descriptions which follow are to be understood as illustrative and exemplary of specific structures, aspects and features within the broad scope of the present invention and not as limiting of such broad scope.

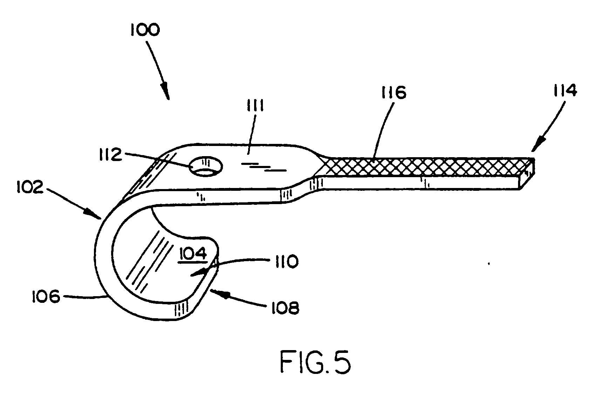

Referring now to Figure 5 a first rod hooking element 100 of the first embodiment of the present invention is provided in a side perspective view. As with all elements of this invention, the material of which this rod hooking element may comprise a high strength material, for example medical grade steel or titanium compounds, which has been found ideally suited for implantation into the human body. This first rod hooking element is designed to hook directly onto a rod, and it is for this reason that it is referred to as rod hooking element 100. This rod hooking element shown in Figure 5 is specifically formed of a thin and flat sheet of metal, the first end 102 of which has been curved to the shape of a hook. The hook has a semi-circular curvature defined by an inner surface 104 and an outer surface 106. The lower portion 108 of the semi-circular hook extends tangentially for a small distance, parallel to the remainder of the element. The curve is semi-circular so that it can cup and hook to the lateral outside surface of a first rod of a dual rod apparatus. The lower extending portion 108 provides addition locking surface 110 against which the rod may be compressed by a locking means described more fully below.

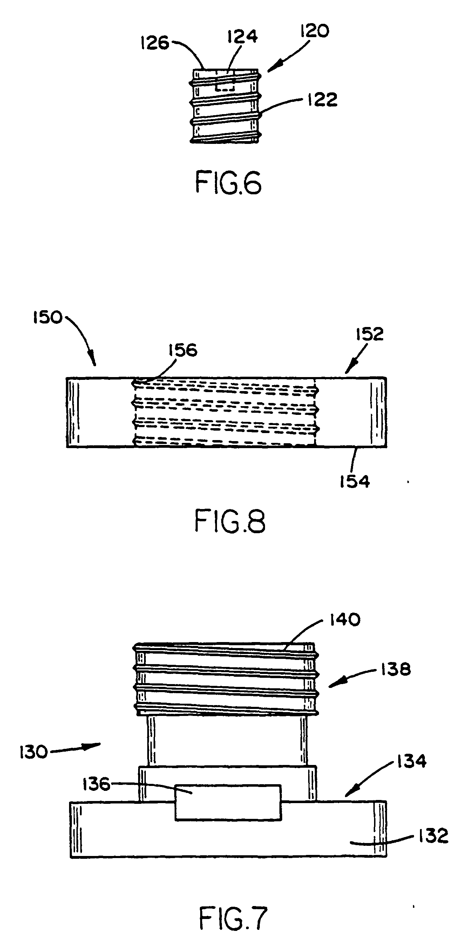

The top portion 111 of the first end 102 comprises a through hole 112 which is threaded and extends from a point on the top surface of the first end 102 which is to adjacent to the initial curvature of the hook portion 102 to the underside of the same. Inasmuch as a set screw (set forth with respect to Figure 6) is to be directed therethrough to lock the rod hooking element 100, and more specifically the first end 102 thereof, to a rod of the implant assembly, the through hole 112 may be angled such that the insertion and subsequent tightening of the set screw drives same toward the inner surface 104 of the hook. Such a preset angle of the through hole orientation enhances the locking strength of the set screw by increasing the cupping surface against which the rod is crushed.

The other end 114 of the rod hooking element 100 comprises a narrowed and flat portion which has a width which is substantially reduced as compared with that of the first rod securing end 102. The thickness and corresponding structural strength of this second end 114 is, however, desirably the same as that of the rod securing end. The top surface 116 of this second narrowed end 114 includes a roughening, for example a diamond knurling, which provides a greater coefficient of static friction to it; this enhanced friction coefficient being desirable for the purposes of compression locking this top surface against another surface.

Referring now to Figure 6, a side view of the set screw 120 which is used to lock the rod to the first end 102 of the rod hooking element 100 (and for applying a compression pressure as described in regards to the second embodiment set forth herein) is provided. The set screw 120 comprises a surface threading 122 which is ideally suited to the threading of the through hole 112. The screw 120 further includes a recess 124 in the top 126 thereof, the recess having an internal conformation which may be engaged by a suitable tool for applying a torsional force thereto, for example a slot for a screwdriver or a hexagonally angled interior wall for an allen wrench.

Referring now to Figures 7 and 8, side view of the post 130 and the top locking nut 150, which together provides the compression coupling of the elements of this embodiment is provided. Specifically referring to Figure 7, the post 130 has a generally cylindrical shape. The base 132 of the post is wider than the upper portions, forming an annular flange 134. This annular flange 134 may include a surface roughening so that it may have an enhanced gripping strength against any surface against which it might be compressed.

The upper portion of the base 132, and the lower portion of the intermediate section of the post 130 includes a passageway 136 which extends transverse to the axis of the post, and which has a substantially rectangular cross-section. This passageway 136 is ideally suited for slidably receiving therethrough the flat extending end 114 of the first rod hooking element 100 (see Figure 5). The height of the passageway 136 is desirably slightly larger than the thickness of the flat extending end 114, but the width of the passageway 136 is desirably the same. These dimensions eliminate any rotation or angulation of the first rod hooking element 100 relative to the post 130.

The upper portion 138 of the post 130 extends upward from the passageway 136 and the intermediate portion, and comprise a threading 140 which is ideally suited for receiving thereon a top locking nut. With reference to Figure 8, the top locking nut 150 comprises a top surface 152 and a bottom surface 154, and an interior threading 156.

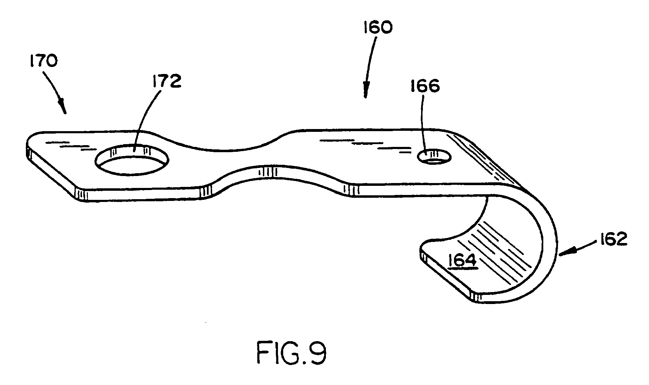

Referring now to Figure 9, the second rod hooking element 160 is provided in a side perspective view. The second rod hooking element 160 includes a rod securing end 162 which is substantially similar to the equivalent feature of the first element 100. More specifically, the rod securing end 162 thereof has a curvate shape and an interior surface 164 for seating against lateral outside surface the second rod of the dual rod apparatus. This curvate end 162 includes a through hole 166 for receiving a set screw 120 for locking the rod to the curvate shaped end.

The other end 170 of this second rod hooking element 160 is substantially wider than the flat end 114 of the first element 100. The undersurface of this end 170 (not shown) may comprise a surface roughening similar to, or at least ideally suited for engaging, the roughened top surface 116 of the first element. This end 170 comprises a through hole 172 which has a width equal to or greater than the width of the upper threaded portion 138, and the intermediate portions of the post 130.

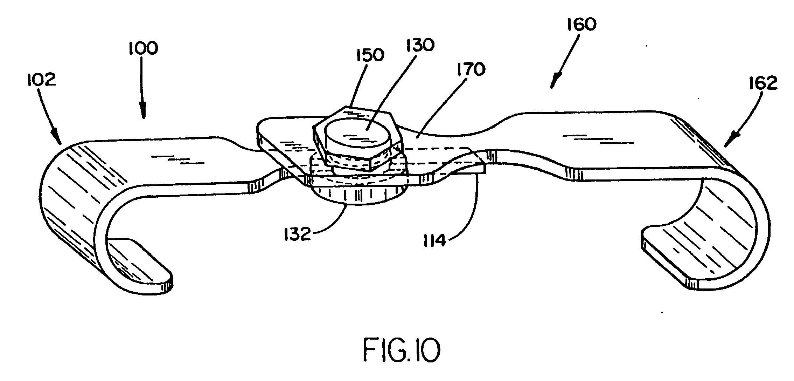

With reference now to Figure 10, in which a fully assembled first embodiment of the present invention is provided in a side perspective view, the method of assembly and the locking of the elements is described. First the proper site for the fixation of the dual rod cross-link device is identified by the surgeon. The site should provide enough space along the rods for engagement of rod securing ends 102 and 162. Once this site is found, and the space separating the rods is cleared of all obstacles (for example, the spinous process) the narrow and flat extending portion 114 of the first rod hooking element 100 is inserted into the passageway 136 of the post 130, such that the roughened top surface 116 of the extending portion 114 is oriented in the direction of the upper section 138 of the post 130.

Once the flat extending portion 114 has been inserted, the upper section 138 of the post 130 is inserted through the hole 172 in the rod coupling flat extending portion 170 of the second rod hooking element 160. The post 130 is inserted upwardly so that the top locking nut 150, and more specifically the threads 156 thereof, may engage the threads 140 of the upper portion 138. Initial tightening of the nut 150 downward on the post 130 causes the bottom surface 154 of the nut to seat against the top surface of the flat extending portion 160. At this point the flat extending portion 114 of the first rod hooking element 100 remains slidable relative to both the post 130 and the second rod hooking element 160. The rod securing portions 102 and 162 may be selectively spread or drawn together by an amount determined solely by the length of the flat extending portion 114 of the first rod hooking element 100. (In addition, it may be understood that inasmuch as the post 130 is not secured to the through hole 172, the post may be rotated, thus angulating the first and second rod hooking elements 100 and 160 relative to one another from their co-linear orientation. While minimal rotation is desired, so as to maximize the contact surfaces of the extending portions 114 and 170, slight angulations may be useful in cases wherein the rods are not locally parallel, but comprise a small convergence or divergence.)

Continued tightening of the nut 150, however, causes the post 130 to be drawn upwards through the hole 172, thereby causing the intermediate section, and the passageway 136 thereof to be similarly drawn upwards. In doing so, the roughened top surface 116 of the narrowed flat extending portion 114 is compressed against the roughened lower surface of the second flat extending portion 170, locking the two together and locking the length of the cross-link.

Once the length of the cross-link is been set, the set screws 120 may be tightened down, thereby locking the rod hooking elements 100 and 160 to the rods themselves.

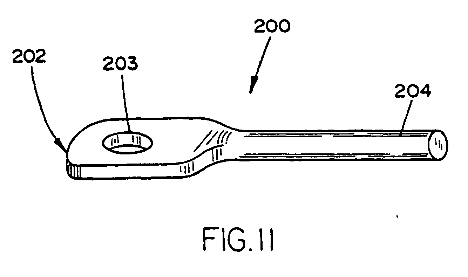

Referring now to Figure 11, a first rod hooking element 200 of the second embodiment of the present invention is provided in a side perspective view. As with the above described embodiment, the rod hooking element 200 comprises a thin and flat sheet of metal, however, in this embodiment, the first end 202 is formed into a loop 204 which fits and may be fastened onto the top pedicle screw. The second end 204 of this first rod hooking element 200 comprises a narrowed cylindrical portion.

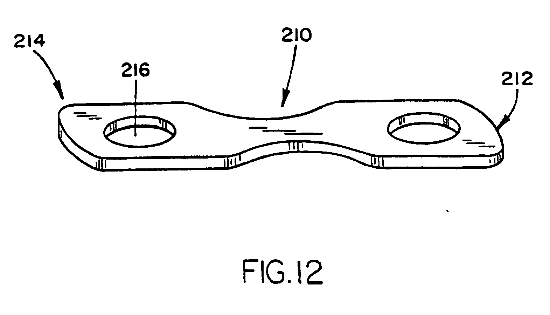

Referring now to Figure 12, the second rod hooking element 210 of this embodiment is shown in a side perspective view. This element includes the same flat looped first end 212 as was set forth with respect to the first rod hooking element 200 of this embodiment. The second end 214 of this second rod hooking element is similar to the second end 170 of the second rod hooking element 160 of the previous embodiment inasmuch as it is flat, and includes a hole 216 through which a threaded post (see below) may be inserted.

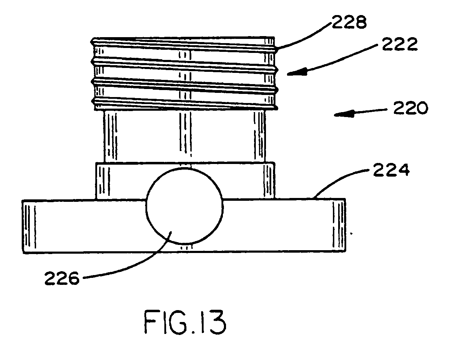

Referring now to Figure 13, a side view of the post 220 which corresponds to this second rod hooking embodiment is provided. As previously described with respect to the post 130 of the first embodiment, the post 220 has a generally cylindrical shape. The base 222 of the post 220 is wider than the upper portions, forming an annular flange. The upper portion of the base 222, and the lower portion of the intermediate section of the post 224 includes a cylindrical passageway 226 which extends transverse to the axis of the post. This passageway 226 is ideally suited for slidably receiving therethrough the cylindrical second end 204 of the first rod hooking element 200. The cylindrical end 204 may slide and rotate therein (such that the length of the device, and the relative angulation of the rod coupling ends may be easily selected).

As set forth above with respect to the first two embodments, the upper portion 222 of the post 220 extends upward from the passageway 216 and the intermediate portion, and comprise a threading 228 which is ideally suited for receiving thereon a top locking nut 150 (as shown in Figure 8).

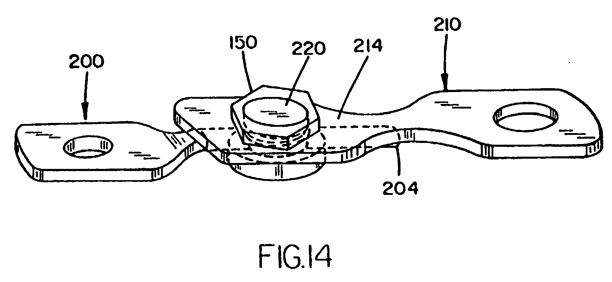

Referring now to Figure 14, in which an assembled device corresponding to this third embodiment is shown in a side perspective view. The cylindrical end 204 of the first rod hooking element 200 is inserted into the cylindrical passageway 226 of the post 220, such that it is axially rotatable and translatable within the passageway 226.

Once the cylindrical second end 204 has been inserted, the upper portion of the post 220 is inserted through the hole 216 in the second end 214 of the second rod hooking element 210. The post 220 is inserted upwardly so that the top locking nut 150, and more specifically the threads 156 thereof, may engage the threads 228 of the upper portion of the post 220. Initial tightening of the nut 150 downward on the post 220 causes the bottom surface 154 of the nut to seat against the top surface of the flat extending portion 214. At this point the cylindrical first end 204 of the first rod hooking element 200 remains slidable and rotatable relative to both the post 220 and the second rod hooking element 210. The first and second rod hooking elements therefore may be spread, angulated and rotated relative to one another.

continued tightening of the nut 150, however, causes the post 220 to be drawn upwards through the hole 216 thereby bringing the cylindrical end 204 into direct contact with the flat end 214, thereby causing the two to crush-lock together. In addition, the compressive force applied to the cylindrical end 204 by the lower surface of the second end 214 of the second element 200 causes the surface of the cylinder 204 to bind to the inner surface of the passageway 226, thereby further enhancing the crush-lock of the device into a rigid configuration.

Once the cross-link has been assembled, the first ends of the rod hooking elements 200 and 210 may be secured to the tops of the pedicle screws. Once the first ends have been secured, the top locking nut 150 is locked down and into rigid position.

While there have been described and illustrated cross-link devices for coupling dual rods of orthopaedic apparatus together and providing enhanced stability thereto, it will be apparent to those skilled in the art that variations and modifications are possible without deviating from the broad spirit and principle of the present invention which shall be limited solely by the scope of the claims appended hereto.

## Claims

1. A variable length cross-link device for use with orthopaedic rod apparatuses having a plurality of rods, comprising:
a first rod hooking element (100) having first and second ends (102,114), and means by which said first end is coupleable to a first rod, said second end comprising a narrow extending portion (114);
a second rod hooking element (160) having first and second ends (162,170), and means by which said first end (162) is coupleable to a second rod, said second end (170) comprising a wide flat extending portion;
first means (130,136) for maintaining said narrow extending portion (114) of said first rod hooking element (100) in slidable relation to the wide flat extending portion (170) of said second rod hooking element (160);
second means (130,150) for selectively compressing a portion of said narrow extending portion (114) against said wide flat extending portion (170) of said second rod hooking element (160) to crush-lock the two elements together, thereby providing for selective variation in the overall length of said cross-link device, **characterized in that**:-
said first means (130,136) also maintains said narrow extending portion (114) of said first rod hooking element (100) in angulable relation to the wide flat extending portion (170) of said second rod hooking element (160)
thereby providing for selective variation in the angulated conformation of said cross link device.

2. A cross-link device as claimed in claim 1, wherein said second rod hooking element (160) has a hole (172) in the wide flat extending portion thereof, and wherein said first and second means (130, 136 and 130, 150) comprise a top locking nut (150) and a post (130), said post (170) having a wide base (132), an intermediate section having a passageway (136) which is transverse to an elongate axis of the post (130), and a threaded upper section (138), such that said narrow extending portion (114) of said first rod hooking element (100) is slidably inserted into said passageway (136), the threaded upper section (138) of said post (130) is inserted through the hole (172) in said wide flat extending portion of said second rod hooking element (160), and the tightening of said top locking nut (150) on said threaded upper section (138) of said post (130) causes said narrow extending portion to be crush-locked against said wide flat extending portion (114).

3. A cross-link device as claimed in claim 1 or claim 2, wherein said narrow extending portion (114) of said first rod hooking element (100) comprises a elongate member having a rectangular cross-sectional conformation.

4. A cross-link device as claimed in claim 3, wherein a top surface (116) of said narrow flat extending portion (114) comprises a roughened surface.

5. A cross-link device as claimed in claim 4, wherein said roughened surface comprises a diamond knurl.

6. A cross-link device as claimed in claim 3, 4 or 5, wherein a bottom surface of said wide flat extending portion (170) comprises a roughened surface.

7. A cross-link device as claimed in claim 6, wherein said roughened surface comprises a diamond knurl.

8. A cross-link device as claimed in any one of the preceding claims, wherein said narrow extending portion of said first rod hooking element comprises a cylindrical conformation.

## Patentansprüche

1. Vernetzungsvorrichtung variabler Länge zum Gebrauch mit orthopädischen Stabgeräten mit einer Mehrzahl von Stäben, umfassend:
ein erstes Stabeinhalcelement (100) mit einem ersten und zweiten Ende (102, 114) und Mitteln, durch die das erste Ende an einen ersten Stab gekoppelt werden kann, wobei das zweite Ende einen schmalen, sich erstreckenden Teil (114) aufweist;
ein zweites Stabeinhakelement (160) mit einem ersten und zweiten Ende (162, 170) und Mitteln, durch die das erste Ende (162) an einen zweiten Stab gekoppelt werden kann, wobei das zweite Ende (170) einen breiten, flachen, sich erstreckenden Teil aufweist;
erste Mittel (130, 136) zum Halten des schmalen, sich erstreckenden Teils (114) des ersten Stabeinhakelements (100) in verschiebbarer Beziehung zu dem breiten, flachen, sich erstreckenden Teil (170) des zweiten Stabeinhakelements (160);
zweite Mittel (130,150) zum selektiven Zusammendrücken eines Teils des schmalen, sich erstreckenden Teils (114) gegen den breiten, flachen, sich erstreckenden Teil (170) des zweiten Stabeinhakelements (160), um die beiden Elemente quetschend aneinander zu verriegeln, wodurch selektive Variation in der Gesamtlänge der Vernetzungsvorrichtung bereitgestellt wird, **dadurch gekennzeichnet, dass**:
das erste Mittel (130, 136) auch den schmalen, sich erstreckenden Teil (114) des ersten Stabeinhakelements (100) in kröpfbarem Verhältnis zu dem breiten, flachen, sich erstreckenden Teil(170) des zweiten Stabeinhakelements (160) hält,
wodurch selektive Variation in der gekröpften Gestalt der Vernetzungsvorrichtung bereitgestellt wird.

2. Vernetzungsvorrichtung nach Anspruch 1, bei der in dem breiten, flachen, sich erstreckenden Teil des zweiten Stabeinhakelements (160) ein Loch (172) vorgesehen ist, und bei der das erste und zweite Mittel (130, 136 und 130, 150) eine obere Befestigungsmutter (150) und einen Pfosten (130) aufweisen, wobei der Pfosten (170) eine breite Basis (132), einen Zwischenabschnitt mit einem Durchgang (136), der quer zu einer Längsachse des Pfostens (130) ist, und einen oberen Gewindeabschnitt (138) aufweist, so dass der schmale, sich erstreckende Teil (114) des ersten Stabeinhakelements (100) verschiebbar in den Durchgang (136) eingeführt wird, der obere Gewindeabschnitt (138) des Pfostens (130) durch das Loch (172) in dem breiten, flachen, sich erstreckenden Teil des zweiten Stabeinhakelements (160) eingeführt wird, und das Festziehen der oberen Befestigungsmutter (150) auf dem oberen Gewindeabschnitt (138) des Pfostens (130) Quetschverriegeln des schmalen, sich erstreckenden Teils an dem breiten, flachen, sich erstreckenden Teil (114) bewirkt.

3. Vernetzungsvorrichtung nach Anspruch 1 oder Anspruch 2, bei der der schmale, sich erstreckende Teil (114) des ersten Stabeinhakelements (100) ein längliches Element mit einer rechteckigen Querschnittsgestalt aufweist.

4. Vernetzungsvorrichtung nach Anspruch 3, bei der eine obere Oberfläche (116) des schmalen, flachen, sich erstreckenden Teils (114) eine angeraute Oberfläche aufweist.

5. Vernetzungsvorrichtung nach Anspruch 4, bei der die angeraute Oberfläche eine Rautenriffelung aufweist.

6. Vernetzungsvorrichtung nach Anspruch 3, 4 oder 5, bei der eine Bodenfläche des breiten, flachen, sich erstreckenden Teils (170) eine angeraute Oberfläche aufweist.

7. Vernetzungsvorrichtung nach Anspruch 6, bei der die angeraute Oberfläche eine Rautenriffelung aufweist.

8. Vernetzungsvorrichtung nach einem der vorhergehenden Ansprüche, bei der der schmale, sich erstreckende Teil des ersten Stabeinhakelements eine zylindrische Gestalt aufweist.

## Revendications

1. Dispositif de liaison à longueur variable destiné à être utilisé avec des appareils à tiges orthopédiques ayant une pluralité de tiges, comportant :
un premier élément d'accrochage pour tige (100) ayant une première extrémité
et une deuxième extrémité (102, 114), et des moyens par le biais desquels ladite première extrémité est en mesure d'être accouplée à une première tige, ladite deuxième extrémité comportant une portion de prolongement étroite (114) ;
un deuxième élément d'accrochage pour tige (160) ayant une première extrémité et une deuxième extrémité (162, 170), et des moyens par le biais desquels ladite première extrémité (162) est en mesure d'être accouplée à une deuxième tige, ladite deuxième extrémité (170) comportant une portion de prolongement plate et large ;
des premiers moyens (130, 136) destinés à maintenir ladite portion de prolongement étroite (114) dudit premier élément d'accrochage pour tige (100) en relation coulissante par rapport à la portion de prolongement plate et large (170) dudit deuxième élément d'accrochage pour tige (160) ;
des deuxièmes moyens (130, 150) destinés à comprimer de manière sélective une portion de ladite portion de prolongement étroite (114) contre ladite portion de prolongement plate et large (170) dudit deuxième élément d'accrochage pour tige (160) en vue de bloquer par écrasement les deux éléments ensemble, pour fournir de ce fait une variation sélective au niveau de la longueur totale dudit dispositif de liaison, **caractérisé en ce que** :
lesdits premiers moyens (130, 136) maintiennent également ladite portion de prolongement étroite (114) dudit premier élément d'accrochage pour tige (100) en une relation angulaire par rapport à la portion de prolongement plate et large (170) dudit deuxième élément d'accrochage pour tige (160)
pour fournir de ce fait une variation sélective au niveau de la conformation angulée dudit dispositif de liaison.

2. Dispositif de liaison selon la revendication 1, dans lequel ledit deuxième élément d'accrochage pour tige (160) a un trou (172) dans la portion de prolongement plate et large de celui-ci, et dans lequel lesdits premiers moyens et lesdits deuxièmes moyens (130, 136 et 130, 150) comportent un écrou de blocage supérieur (150) et un goujon (130), ledit goujon (170) ayant une base large (132), une section intermédiaire ayant une voie de passage (136) qui est transversale par rapport à un axe allongé du goujon (130), et une section supérieure filetée (138), de telle manière que ladite portion de prolongement étroite (114) dudit premier élément d'accrochage pour tige (100) est insérée de manière coulissante dans ladite voie de passage (136), la section supérieure filetée (138) dudit goujon (130) est insérée au travers du trou (172) dans ladite portion de prolongement plate et large dudit deuxième élément d'accrochage pour tige (160), et le serrage dudit écrou de blocage supérieur (150) sur ladite section supérieure filetée (138) dudit goujon (130) amène ladite portion de prolongement étroite à être bloquée par écrasement contre ladite portion de prolongement plate et large (114).

3. Dispositif de liaison selon la revendication 1 ou la revendication 2, dans lequel ladite portion de prolongement étroite (114) dudit premier élément d'accrochage pour tige (100) comporte un organe allongé ayant une conformation en coupe rectangulaire.

4. Dispositif de liaison selon la revendication 3, dans lequel une surface supérieure (116) de ladite portion de prolongement plate et étroite (114) comporte une surface rugueuse.

5. Dispositif de liaison selon la revendication 4, dans lequel ladite surface rugueuse comporte un moletage croisé oblique.

6. Dispositif de liaison selon la revendication 3, la revendication 4 ou la revendication 5, dans lequel une surface inférieure de ladite portion de prolongement plate et large (170) comporte une surface rugueuse.

7. Dispositif de liaison selon la revendication 6, dans lequel ladite surface rugeuse comporte un moletage croisé oblique.

8. Dispositif de liaison selon l'une quelconque des revendications précédentes, dans lequel ladite portion de prolongement étroite dudit premier élément d'accrochage pour tige comporte une conformation cylindrique.
